# FASCICULE DE BREVET EUROPEEN

(11) **EP 3 359 185 B1**
(45) Date de publication et mention de la délivrance du brevet: **06.04.2022**
(21) Numéro de dépôt: 16794685.4
(22) Date de dépôt: 07.10.2016
(51) Int. Cl.: A61K 39/00, A61P 35/00, A61P 37/04

(54) **COMPOSITION ANTI-TUMORALE**
ANTITUMORALE ZUSAMMENSETZUNG
ANTI-TUMORAL COMPOSITION

(30) Priorité: 08.10.2015 FR 1559589; 30.09.2016 FR 1659450
(43) Date de publication de la demande: 15.08.2018
(73) Titulaire: Limacher, Jean-Marc, 67150 Matzenheim (FR)
(72) Inventeur: Limacher, Jean-Marc, 67150 Matzenheim (FR)
(74) Mandataire: Oudin, Stéphane
(86) Numéro de dépôt international: PCT/FR2016/052598
(87) Numéro de publication internationale: WO 2017/060650

(56) Documents cités:
- WO-A1-98/04705
- WO-A1-2004/026337
- WO-A1-2012/017210
- WO-A1-2015/069571
- WO-A2-2015/095811
- US-A1- 2014 271 724
- A Q BUTT ET AL: "Immunosuppressive networks and checkpoints controlling antitumor immunity and their blockade in the development of cancer immunotherapeutics and vaccines", ONCOGENE, vol. 33, no. 38, 21 octobre 2013 (2013-10-21), pages 4623-4631, XP055257733, GB ISSN: 0950-9232, DOI: 10.1038/onc.2013.432
- ESPENSCHIED JONATHAN ET AL: "CTLA-4 blockade enhances the therapeutic effect of an attenuated poxvirus vaccine targeting p53 in an established murine tumor model", THE JOURNAL OF IMMUNOLOGY, THE AMERICAN ASSOCIATION OF IMMUNOLOGISTS, US, vol. 170, no. 6, 15 mars 2003 (2003-03-15) , pages 3401-3407, XP002443898, ISSN: 0022-1767
- MALA CHAKRABORTY ET AL: "The combined activation of positive costimulatory signals with modulation of a negative costimulatory signal for the enhancement of vaccine-mediated T-cell responses", CANCER IMMUNOLOGY, IMMUNOTHERAPY, SPRINGER, BERLIN, DE, vol. 56, no. 9, 23 février 2007 (2007-02-23), pages 1471-1484, XP019514192, ISSN: 1432-0851, DOI: 10.1007/S00262-007-0291-6
- T. N. SCHUMACHER ET AL: "Neoantigens in cancer immunotherapy", SCIENCE, vol. 348, no. 6230, 3 avril 2015 (2015-04-03), pages 69-74, XP055211712, ISSN: 0036-8075, DOI: 10.1126/science.aaa4971
- JASON RICE ET AL: "DNA vaccines: precision tools for activating effective immunity against cancer", NATURE REVIEWS. CANCER, vol. 8, no. 2, février 2008 (2008-02), pages 108-120, XP055257615, GB ISSN: 1474-175X, DOI: 10.1038/nrc2326

## Description

### Domaine technique

La présente invention concerne une composition pharmaceutique caractérisée en ce qu'elle comprend au moins une séquence d'acide nucléique, cette séquence d'acides nucléiques n'étant pas incluse dans un génome viral ni encapsulée dans une particule virale, codant au moins un néoantigène spécifique d'une cellule cancéreuse, au moins une particule virale de type poxvirus non-recombinant, et un anticorps anti-CTLA4.

### Technique antérieure

Traditionnellement, dans le cadre des pathologies tumorales, les traitements consistent en l'utilisation de composés chimiques ou biologiques (chimiothérapie), de rayonnements (radiothérapie) et/ou de la chirurgie. Bien que d'immenses progrès aient été observés dans les dernières années avec des thérapies ciblées mettant notamment en œuvre des anticorps monoclonaux ou d'autres molécules ciblant des récepteurs spécifiques, on observe toujours une mortalité importante et d'autres voies thérapeutiques sont toujours envisagées afin de faire diminuer la mortalité associée et/ou les effets secondaires liés à ces pathologies et à leurs traitements.

Parmi les nouvelles voies thérapeutiques envisagées, on peut notamment citer la thérapie génique et l'immunothérapie. L'idée d'immuniser contre des cellules cancéreuses préexistantes est basée sur l'observation que les cellules cancéreuses expriment des protéines mutées, des protéines glycosilées de façons aberrantes ou, dans le cas de tumeurs induites par infection virale, des protéines virales. Au sein de la tumeur, l'expression de ces protéines n'entraine pas la production d'une réponse immunitaire efficace. En effet, la tumeur est notamment capable de créer un microenvironnement qui empêche l'initiation d'une réponse immunitaire ciblant ces antigènes et/ou les cellules qui les expriment. De plus, même si une réponse immunitaire est induite, elle n'arrive pas toujours à être efficace en raison de l'anergie qui règne dans la tumeur. Il a donc été postulé qu'il est plus efficace, en termes de réponse immunitaire générée d'injecter les antigènes associés aux cellules tumorales en une zone où le système immunitaire est actif.

Parmi les antigènes associés aux tumeurs, on peut également citer les néoantigènes tumoraux issus de l'accumulation de mutations dans une population cellulaire ce qui conduit à la cancérisation. Ces mutations peuvent résulter par exemple de l'exposition aux rayons ultra-violets ou bien des substances cancérigènes du tabac ou d'autre nature. Certaines sont directement impliquées dans la cancérogenèse, d'autres sont simplement en situation de « passager » mais toutes, du fait du changement d'acides aminés dans la séquence d'une protéine ont le potentiel d'être considérées par le système immunitaire comme différentes du soi et donc antigéniques.

Dans le cadre du traitement du cancer, la thérapie génique a été utilisée selon trois axes principaux. Le premier a consisté à injecter sur le site de la tumeur des gènes codant des molécules immunostimulatrices aptes à lever l'inhibition locale du système immunitaire. Par exemple, des gènes codant des interleukines ont été injectés directement à l'intérieur des tumeurs. La production locale d'interleukines était supposée permettre l'activité normale du système immunitaire et la destruction des cellules cancéreuses. Toutefois, les résultats obtenus avec ce type de stratégie ont été décevants.

Un deuxième axe de recherche consiste à injecter dans les cellules cancéreuses des gènes codant des molécules cytotoxiques ou entrainant la production directe ou indirecte de molécules cytotoxiques. Cette stratégie est censée permettre la destruction des cellules tumorales et éventuellement le relargage d'antigènes dans l'organisme permettant ainsi l'activation du système immunitaire.

Le dernier axe de recherche a consisté à utiliser des acides nucléiques codant des antigènes associés aux tumeurs pour vacciner les patients. En effet, la production de l'antigène par les cellules du patient, plutôt que leur injection in situ, permet d'envisager une meilleure présentation de l'antigène plus conforme à ce qui se passe au niveau des cellules cancéreuses.

US2014/271724A1 divulgue une composition comprenant (a) un vecteur exprimant un antigène spécifique de cellules cancéreuses fusionné à un anticorps anti-CTLA4 et (b) un vecteur exprimant la protéine FAP. Les vecteurs (a) et (b) sont indépendamment sélectionnés parmi un acide nucléique, un vecteur adénovirus ou poxvirus.

WO2015/095811A2 divulgue une composition comprenant un peptide d'une cellule cancéreuse et un inhibiteur de checkpoint immunitaire. WO2015/095811A2 divulgue également l'utilisation de poxvirus exprimant un antigène spécifique de cellules cancéreuses ainsi que d'un anticorps anti-CTLA4. WO2015/095811A2 divulgue des antigènes de HPV pour le traitement de lésions malignes induites par HPV.

Toutefois, la réponse anti tumorale observée dans le cadre de ces différents traitements, n'est pas beaucoup meilleure que les réponses cliniques observées dans le cadre de traitements classiques.

Il est par conséquent souhaitable de disposer de nouveaux produits et/ou de nouvelles méthodes permettant un contrôle prolongé du volume tumoral et l'augmentation du taux de survie des patients traités.

### Résumé de l'invention

La présente invention concerne une composition pharmaceutique caractérisée en ce qu'elle comprend au moins une séquence d'acide nucléique, cette séquence d'acides nucléiques n'étant pas incluse dans un génome viral ni encapsulée dans une particule virale, codant au moins un néoantigène spécifique d'une cellule cancéreuse, au moins une particule virale de type poxvirus non-recombinant, et un anticorps anti-CTLA4.

La combinaison spécifique de ces trois composants permet d'obtenir une réponse immunitaire ciblée contre le ou les néoantigènes codés par la séquence d'acide nucléique. Le poxvirus permet à la fois d'améliorer la transfection de la séquence d'acide nucléique et d'améliorer la qualité de la réponse immune générée contre le ou les néoantigènes. L'anticorps anti-CTLA-4 permet également d'améliorer la réponse immunitaire contre le ou les néoantigènes. L'utilisation d'une séquence d'acide nucléique permet d'envisager une production rapide et personnalisée de la séquence codant pour le ou les néoantigènes. En effet, les techniques actuelles permettent de séquencer intégralement le patrimoine génétique des cellules cancéreuses d'un patient et d'identifier les mutations spécifiques par comparaison constitutionnel du patient. Si ces mutations sont non-synonymes, appartiennent à des séquences codantes et exprimées par la tumeur, et sont de par leurs caractéristiques supposées immunogènes alors ces dernières peuvent être utilisées pour induire l'immunisation contre la tumeur.

Les mutations dans le génome des tumeurs et les néoantigènes qui en résultent sont une combinatoire unique restreinte à la tumeur d'un patient donné, voire aux clones qui composent cette tumeur.

L'utilisation des néoantigènes comme moyen d'immunisation d'un patient contre ses propres cellules tumorales est un concept nouveau. Sa mise en œuvre est rendue compliquée par la nécessité de produire en un temps court une préparation pharmaceutique personnalisée correspondant aux néoantigènes portés par la tumeur d'un patient donné.

Jusqu'à ce jour l'immunisation thérapeutique en oncologie a fait appel à deux grandes catégories de produits. La première consiste à administrer, le plus souvent par injection, des peptides ou protéines supposés être des antigènes présents dans la tumeur, le tout en présence de substances adjuvantes dont l'emploi soulève parfois des réticences. La deuxième consiste en l'administration d'acides nucléiques codant pour des antigènes supposés être présents dans la tumeur mais correspondant le plus souvent à des protéines exprimées préférentiellement par la tumeur mais qui ne sont pas nécessairement différentes de la version non-tumorale et ne satisfont pas nécessairement à la définition d'un néoantigène.

De ces deux classes de produits les immunothérapies par thérapie génique, en particulier celles reposant sur l'emploi de vecteurs viraux, ont montré de façon reproductible une efficacité significative dans le traitement des tumeurs. Les immunothérapies par thérapie génique, a fortiori celles à base virale, sont naturellement perçues par le système immunitaire comme un signal de danger. Elles sont plus immunogènes et ne nécessitent en principe pas l'emploi d'adjuvants. La composante virale, en particulier s'il s'agit d'un poxvirus, est source d'une réponse immunitaire innée favorable au développement d'une réponse immunitaire adaptative contre les antigènes exprimés.

La conception et la production pharmaceutique d'un virus génétiquement modifié exprimant les séquences codant pour un ou plusieurs néoantigènes présents dans la tumeur d'un patient est une option techniquement faisable mais cependant il s'agit d'une opération longue et coûteuse très difficilement généralisable.

A l'inverse la production de plasmides, ou d'acides nucléiques linéaires fermés double-brins, ou d'autres vecteurs d'expression génétiques codant pour un ou plusieurs néoantigènes est une opération simple, relativement peu onéreuse, réalisable en quelques semaines tout au plus.

Le principe de co-injection d'un acide nucléique codant pour un ou plusieurs néoantigènes et d'un virus, en particulier d'un poxvirus, est de nature à permettre la production dans un temps court d'une préparation pharmaceutique personnalisée correspondant aux néoantigènes portés par la tumeur d'un patient tout en bénéficiant de la présence d'un virus apportant le signal de danger utile au développement de la réponse immunitaire.

De surcroît le poxvirus de par la composante phospholipidique de sa membrane est de nature à favoriser la pénétration de l'acide nucléique dans les cellules présentes au voisinage du point d'injection, et par conséquent l'expression des protéines antigéniques pour lesquelles code l'acide nucléique.

La séquence d'acide nucléique dans le cas d'un vecteur d'expression à ADN, favorise la reconnaissance par des senseurs spécifiques de l'ADN double brin dans le cytoplasme des cellules dans lesquelles ces vecteurs ont pénétré. Cette reconnaissance est à l'origine d'une réponse biologique favorable à la réponse immunitaire.

La séquence d'acide nucléique et le poxvirus ont donc des voies d'actions différentes qui se complètent et permettent d'obtenir une meilleure réponse.

Les anticorps anti-CTLA4 sont des inhibiteurs d'un point de contrôle négatif de la réponse immunitaire. Ils ont jusque-là été administrés par voie systémique, intraveineuse, avec une efficacité significative, tout au moins dans le traitement du mélanome malin cutané et le cancer du poumon non à petites cellules, mais au prix d'effets indésirables non négligeables.

La co-administration d'un anticorps ou fragment d'anticorps anti-CTLA4, est de nature à renforcer la réponse immunitaire induite par une immunothérapie, en particulier la combinaison d'un vecteur d'expression génétique et d'un poxvirus. Pour cela des doses réduites à 1/20ème ou moins de la dose systémique usuelle sont supposées suffisantes. A ces doses et après co-injection la concentration dans le ganglion de drainage du point d'injection est supposée être plus élevée que par voie systémique alors que la concentration systémique elle-même est grandement réduite, le risque d'effet indésirable également.

Dans le cadre de la présente invention, le terme « séquence d'acide nucléique » fait référence à une séquence d'acide nucléique codante de type ADN ou ARN. Cette séquence d'acides nucléiques n'est pas incluse dans un génome viral, ni encapsulée dans une particule virale.

Selon un mode de réalisation de l'invention, ladite séquence d'acide nucléique est sélectionnée dans le groupe comprenant les plasmides et les vecteurs d'ADN linéaire ou d'ARN.

Selon un mode de réalisation de l'invention, ladite séquence d'acide nucléique est nue c'est-à-dire qu'elle n'est pas associée à des molécules favorisant sa pénétration dans des cellules eucaryotes comme, par exemple, des polymères cationiques, des protéines virales, des lipides et/ou des liposomes.

Selon un mode de réalisation préféré de l'invention, ladite séquence d'acide nucléique est un acide nucléique linéaire fermé double-brin. De plus, les acides nucléique linéaires fermés par leur mode de production s'accompagnent de très peu de résidus de production, en particulier bactériens, ce qui permet d'éviter de lourdes étapes de purification et permet une utilisation plus rapide et moins onéreuse.

Les acides nucléiques linéaires fermés sont bien connus de l'homme du métier, leur structure, leurs fonctions et les procédés permettant leur production sont notamment décris dans les documents CN103080337, EP2601312, GB201013153, JP2013535210, US2013216562, WO12017210, AU2010209532, CA2751130, CN102301010, DK2391731, EA021069, EA201101141, EP2391731, EP2612925, ES2400890, GB200901593, HK1159693, IL213930, IN05006CN2011, JP2012516147, KR20110107846, MX2011007937, NZ594004, SG173102, US2012282283, US9109250, WO10086626.

Dans le cadre de la présente invention, le terme « néoantigène associé à une cellule cancéreuse » fait référence à une protéine exprimée par la cellule cancéreuse sous une forme mutée par comparaison avec la séquence constitutionnelle du patient.

Selon un mode de réalisation préféré de l'invention, l'anticorps anti-CTLA-4 et préférentiellement choisi dans le groupe comprenant l'ipilimumab et le tremelimumab. Il peut également s'agir d'un fragment d'anticorps dirigé contre CTLA-4 ou de toute molécule contenant un paratope dirigé spécifiquement contre le CTLA4. Par souci de clarté, il est précisé que le terme « anticorps anti-CTLA4 » ne concerne pas les acides nucléiques codant un antidorps anti-CTLA4.

Selon un mode de réalisation préféré, ladite séquence d'acide nucléique comprend en outre les éléments de régulation assurant l'expression du ou des néoantigènes spécifiques d'une cellule cancéreuse dans les cellules eucaryotes.

Selon un mode de réalisation préféré, les éléments de régulation assurant l'expression du ou des néoantigènes associé à une cellule cancéreuse dans les cellules eucaryotes comportent un promoteur de la transcription du gène et une région d'initiation de la traduction dans les cellules hôtes. Ladite séquence d'acide nucléique peut comprendre plusieurs séquences codant chacune un néoantigène différent associé aux tumeurs. Dans le cas d'une pluralité de néoantigènes, les séquences codant ces derniers peuvent être placées sous la dépendance d'éléments de régulation identiques ou différents.

Selon un mode de réalisation préféré, le poxvirus est vivant ou tué.

Le poxvirus ne comprend pas de séquences hétérologues.

Selon un mode de réalisation préféré, le poxvirus dérive d'un virus de la vaccine, d'un canaripox ou d'un fowlpox.

Selon un mode de réalisation préféré, ledit poxvirus dérive d'un virus de la vaccine sélectionné parmi les souches Copenhague, Wyeth et Ankara modifiée (MVA).

Selon un mode de réalisation préféré, ledit poxvirus dérive d'un virus de la vaccine de la souche Copenhague. Selon un mode de réalisation préféré, ledit poxvirus dérive d'un virus de la vaccine de la souche MVA.

Dans le cadre de la présente invention, le terme « dérive » entend signifier que ledit virus appartient à ladite souche.

La présente invention concerne également une composition selon l'invention comprenant en outre un support pharmaceutiquement acceptable permettant son administration par injection à l'homme ou à l'animal.

Selon un mode de réalisation préféré de l'invention, ledit support pharmaceutiquement acceptable permet l'injection de ladite composition via un dispositif d'injection sans aiguilles.

Dans le cadre de la présente invention, le terme « dispositif d'injection sans aiguilles » fait référence à un dispositif pour les injections intradermiques, sous-cutanées ou intramusculaires, d'un principe actif liquide à usage thérapeutique en médecine humaine ou vétérinaire mettant en œuvre un jet de liquide sous pression pour pénétrer les tissus. Le liquide peut être plus ou moins visqueux, un mélange de liquide, ou un gel.

L'administration d'acides nucléiques nécessite en général l'emploi d'une technique d'électroporation couplée à l'injection. L'injection sous pression par un système sans aiguille est une autre solution. Elle a aussi l'avantage du fait de l'injection sous pression d'assurer une meilleure diffusion dans les espaces tissulaires. Son emploi autorise aussi si nécessaire la préparation extemporanée d'un mélange de produits à injecter. L'emploi d'un système d'injection sous pression sans aiguille est de nature à favoriser une meilleure pénétration tissulaire qu'avec une aiguille 5 classique et donc la transfection d'un plus grand nombre de cellules. L'objectif est de favoriser ainsi la réponse immunitaire contre les néoantigènes exprimés par la séquence d'acide nucléique.

La présente invention concerne également un ensemble departies comprenant une séquence d'acide nucléique codant au moins un néoantigène spécifique d'une cellule cancéreuse, au moins un poxvirus, et un Anticorps ou fragment d'anticorps dirigé contre CTLA4.

Ledit ensemble de parties peut notamment être utilisé pour préparer extemporanément une composition selon l'invention.

La présente invention concerne également l'utilisation d'une composition selon l'invention pour le traitement d'un cancer ou d'une tumeur.

La présente invention concerne également l'utilisation d'une composition selon l'invention pour le traitement d'un cancer du col de l'utérus, de la sphère ORL ou de toute autre tumeur induite par le virus HPV, d'un cancer du foie lié au virus de l'hépatite chronique B ou C, ou d'un cancer exprimant la protéine MUC1.

### Description des modes de réalisation

Selon un mode de réalisation préféré de l'invention, les cellules cancéreuses d'un patient à traiter sont analysées à partir d'une biopsie ou pièce opératoire.. L'ADN et l'ARN des cellules cancéreuses, ainsi que l'ADN constitutionnel issu d'une prise de sang, sont extraits et séquencés.

La séquence ADN des cellules saines est comparée à la séquence ADN des cellules cancéreuses et les mutations somatiques comprises dans des régions codantes et exprimées sont identifiées.

Les séquences mutées codantes et exprimées sont traitées à l'aide d'algorithmes permettant de définir pour chaque patients quelles mutations sont potentiellement les plus antigéniques 10 et peuvent servir de cible. Ces séquences mutées, non synonymes, codantes, exprimées, spécifiques des cellules cancéreuses du patient, appelées néoantigènes, sont ensuite générées par synthèse d'ADN ou d'ADN puis mises sous la forme d'un vecteur d'expression (plasmide ou acide nucléique linéaire fermé ou ARN) . Selon un mode de réalisation préféré de l'invention, en utilisant la méthode décrite dans la demande de brevet EP2391731, La séquence d'acide nucléique codant un ou plusieurs néoantigènes associés à la tumeur du patient est placée, à l'intérieur de l'acide nucléique linéaire fermé, sous la dépendance des séquences nécessaires à son expression en cellules eucaryotes. Ainsi, ladite séquence est préférentiellement placée en aval d'un promoteur d'expression eucaryote et en amont d'une séquence de terminaison de la transcription. II peut s'agir d'un promoteur eucaryote fort et en particulier d'un promoteur précoce du CMV. Le promoteur est soit d'origine virale, soit d'origine cellulaire. Comme promoteur viral autre que CMV on peut citer le promoteur précoce ou tardif du virus SV40 ou le promoteur LTR du virus du Sarcome de Rous. Comme promoteur cellulaire, on peut citer le promoteur d'un gène du cytosquelette, tel que par exemple le promoteur de la desmine, ou encore le promoteur de l'actine. Préférentiellement, un intron pourra être intégré à l'intérieur de ladite séquence codant un ou plusieurs néoantigènes associés à la tumeur du patient. En effet, certains introns sont connus pour augmenter la transcription des séquences d'acides nucléiques.

Le Poxvirus utilisé dans la composition est préférentiellement un virus MVA. Ce dernier dérive de la souche Ankara du virus de la vaccine par passages successifs sur des cellules d'embryons de poulet. Ces différents passages ont entrainé l'atténuation de ce virus qui a ainsi pu être utilisé dans les dernières campagnes de vaccination contre la variole.

La description des différentes souches de MVA, les méthodes permettant d'insérer éventuellement des gènes exogènes dans son génome ainsi que les méthodes de production, et de purification de ce virus sont notamment accessibles dans les documents WO0168820, WO0242480, WO03008533, WO03048184, WO03053463, WO03054175, WO03088994, WO03097675, WO03097844, WO03097845, WO03097846, WO04048582, WO04048606, 15WO05054484, WO06089690, WO08028665, WO08045346, WO08131926, WO08131927, WO08138533, WO09052328, WO09152969, WO10057650, WO10060632, WO10102822, WO11042180, WO11092029, WO12010280, WO12048817, WO12059243, WO13083254, WO13189611, WO14019718, WO14037124, WO14062778, WO14063832, WO9813500, WO9915692.

Enfin, la composition selon l'invention comprendra préférentiellement un anticorps ou fragment d'anticorps anti-CTLA4, encore plus préférentiellement de l'ipilimumab. Dans le cadre de la présente invention, le terme anticorps désigne également les anticorps bispécifiques comprenant au moins un paratope spécifique du CTLA-4.

De façon préférée, la composition selon l'invention comprend en outre un support pharmaceutiquement acceptable. Dans le cadre de la présente invention, le terme "support pharmaceutiquement acceptable" entend désigner tous les supports, solvants, diluants, excipients, adjuvants, milieux de dispersion, et analogues, compatibles avec une administration pharmaceutique.

La composition à utiliser dans l'invention est convenablement tamponnée afin d'être appropriée pour une utilisation humaine à un pH physiologique ou légèrement basique.

La composition selon l'invention peut être administrée au patient par une variété de modes d'administration comme par exemple les voies sous-cutanée, intradermique, intramusculaire, intraveineuse, intrapéritonéale, intratumorale, intravasculaire, intra-artérielle.

Les injections peuvent être faites avec des seringues et des aiguilles conventionnelles, mais préférentiellement via un dispositif d'injection sans aiguilles de types bioject(r).

L'administration de la composition selon l'invention peut avoir lieu en dose unique ou répétée après un certain intervalle de temps allant d'un jour à un an. Préférentiellement l'administration aura lieu de façon hebdomadaire sept fois de suite puis ensuite une fois toutes les trois semaines.

Le dosage approprié peut être adapté en fonction de divers paramètres, en particulier le mode d'administration, la composition utilisée, l'âge, la santé, et le poids de l'organisme hôte, la nature et l'étendue des symptômes, le type de traitement associé, la fréquence du traitement.

L'homme du métier est capable de déterminer les quantités adaptées de chaque élément à l'intérieur de la composition selon l'invention. A titre d'exemple, le poxvirus peut être utilisé à une quantité comprise entre 10⁴ à 10⁹ pfu, la séquence d'acide nucléique nue en une quantité comprise entre 10 µg et 20 mg, et l'anticorps anti-CTLA4 en une quantité comprise entre 10 ng et 20 mg par injection.

De façon préférentielle, la composition selon l'invention peut être utilisée en conjonction avec la radiothérapie, la chimiothérapie la chirurgie et/ou d'autres produits d'immunothérapie tels les anticorps anti PD1 et 5 anti-PDL1, ou la combinaison de plusieurs de ces traitements à la fois.

## Revendications

1. Composition pharmaceutique **caractérisée en ce qu'**elle comprend au moins une séquence d'acide nucléique, cette séquence d'acides nucléiques n'étant pas incluse dans un génome viral ni encapsulée dans une particule virale, codant au moins un néoantigène spécifique d'une cellule cancéreuse, au moins une particule virale de type poxvirus non-recombinant, et un anticorps anti-CTLA4.

2. Composition selon la revendication l, **caractérisée en ce que** ladite séquence d'acide nucléique nue comprend en outre les éléments de régulation assurant l'expression dudit ou desdits antigène(s) spécifique(s) d'une cellule cancéreuse dans les cellules eucaryotes.

3. Composition selon l'une des revendications précédentes, **caractérisée en ce que** ladite séquence d'acide nucléique est choisie dans le groupe comprenant les plasmides, les vecteurs d'ADN linéaire fermé, ou encore des molécules d'ARN.

4. Composition selon l'une des revendications précédentes, **caractérisée en ce que** ladite séquence d'acide nucléique est un acide nucléique linéaire fermé.

5. Composition selon l'une des revendications 2 à 4, **caractérisée en ce que** les éléments de régulation assurant l'expression du ou des néoantigènes spécifiques d'une cellule cancéreuse dans les cellules eucaryotes comportent un promoteur de la transcription du gène et une région d'initiation de la traduction dans les cellules hôtes.

6. Composition selon l'une des revendications précédentes **caractérisée en ce que** le poxvirus est vivant ou tué.

7. Composition selon l'une des revendications précédentes **caractérisée en ce que** la particule de poxvirus dérive d'un virus de la vaccine, d'un canaripox ou d'un fowlpox.

8. Composition selon l'une des revendications précédentes, **caractérisée en ce qu'**elle comporte un support pharmaceutiquement acceptable permettant son administration par injection à l'homme ou à l'animal.

9. Composition selon la revendication précédente **caractérisée en ce que** ledit support pharmaceutiquement acceptable permet l'injection de ladite composition via un dispositif d'injection sans aiguilles.

10. Composition selon l'une des revendications précédentes, pour une utilisation dans le traitement d'un cancer ou d'une tumeur.

11. Composition selon la revendication 10, pour une utilisation dans le traitement d'un cancer du col de l'utérus, de la sphère ORL ou de toute autre tumeur induite par le virus HPV, d'un cancer du foie lié au virus de l'hépatite chronique C, ou d'un cancer exprimant la protéine MUC1.

## Patentansprüche

1. Pharmazeutische Zusammensetzung, **dadurch gekennzeichnet, dass** sie mindestens eine Nukleinsäurensequenz umfasst, wobei diese Nukleinsäurensequenz nicht in einem viralen Genom noch in einem viralen Partikel eingekapselt ist, die mindestens ein spezifisches Neoantigen einer Krebszelle, mindestens einen viralen Partikel vom Typ Poxvirus und einen Anti-CTLA4-Antikörper codiert.

2. Zusammensetzung nach Anspruch 1, **dadurch gekennzeichnet, dass** die nackte Nukleinsäurensequenz ferner die Regulationselemente umfasst, die die Expression des oder der spezifischen Antigens/Antigene einer Krebszelle in den eukaryotischen Zellen sichern.

3. Zusammensetzung nach einem der vorangehenden Ansprüche, **dadurch gekennzeichnet, dass** die Nukleinsäurensequenz aus der Gruppe ausgewählt ist, die die Plasmide, die Vektoren geschlossener linearer DNA oder auch RNA-Moleküle umfasst.

4. Zusammensetzung nach einem der vorangehenden Ansprüche, **dadurch gekennzeichnet, dass** die Nukleinsäurensequenz eine geschlossene lineare Nukleinsäure ist.

5. Zusammensetzung nach einem der Ansprüche 2 bis 4, **dadurch gekennzeichnet, dass** die Regulationselemente, die die Expression des oder der spezifischen Neoantigens/Neoantigene einer Krebszelle in den eukaryotischen Zellen sichern, einen Promoter der Transkription des Gens und eine Initiationsstelle der Translation in den Hostzellen aufweisen.

6. Zusammensetzung nach einem der vorangehenden Ansprüche, **dadurch gekennzeichnet, dass** das Poxvirus lebt oder abgetötet ist.

7. Zusammensetzung nach einem der vorangehenden Ansprüche, **dadurch gekennzeichnet, dass** der Poxviruspartikel von einem Virus des Impfstoffs, einem Canaripox oder einem Fowlpox abgeleitet ist.

8. Zusammensetzung nach einem der vorangehenden Ansprüche, **dadurch gekennzeichnet, dass** sie einen pharmazeutisch akzeptablen Träger aufweist, der erlaubt, sie dem Menschen oder dem Tier durch Injektion zu verabreichen.

9. Zusammensetzung nach vorangehendem Anspruch, **dadurch gekennzeichnet, dass** der pharmazeutisch akzeptable Träger die Injektion der Zusammensetzung über eine Injektionsvorrichtung ohne Nadeln erlaubt.

10. Zusammensetzung nach einem der vorangehenden Ansprüche für eine Verwendung zur Behandlung eines Krebses oder eines Tumors.

11. Zusammensetzung nach Anspruch 10 für eine Verwendung zur Behandlung eines Krebses des Gebärmutterhalses, des HNO-Bereichs oder jedes anderen Tumors, der vom HPV-Virus induziert ist, eines Leberkrebses in Verbindung mit dem Virus der chronischen Hepatitis C oder eines Krebses, der das Protein MUCl exprimiert.

## Claims

1. Pharmaceutical composition **characterised in that** it comprises at least one nucleic acid sequence, this nucleic acid sequence not being included in a viral genome nor encapsulated in a viral particle, encoding at least one specific neoantigen of a cancer cell, at least one non-recombinant poxvirus type viral particle, and an anti-CTLA4 antibody.

2. Composition according to claim 1, **characterised in that** said bare nucleic acid sequence further comprises the regulatory elements ensuring the expression of said specific neoantigen(s) of a cancer cell in eukaryotic cells.

3. Composition according to one of the preceding claims, **characterised in that** said nucleic acid sequence is chosen from the group comprising plasmids, closed linear DNA vectors, or else RNA molecules.

4. Composition according to one of the preceding claims, **characterised in that** said nucleic acid sequence is a closed linear nucleic acid sequence.

5. Composition according to one of claims 2 to 4, **characterised in that** the regulatory elements ensuring the expression of the specific neoantigen(s) of a cancer cell in eukaryotic cells include a gene transcription promoter and a translation initiation region in the host cells.

6. Composition according to one of the preceding claims **characterised in that** the poxvirus is live or killed.

7. Composition according to one of the preceding claims **characterised in that** the poxvirus particle derives from a vaccinia virus, a canarypox or a fowlpox.

8. Composition according to one of the preceding claims, **characterised in that** it includes a pharmaceutically acceptable carrier allowing the administration thereof by injection to humans or animals.

9. Composition according to the preceding claim **characterised in that** said pharmaceutically acceptable substrate allows the injection of said composition via a needleless injection device.

10. Composition according to one of the preceding claims, for use in the treatment of a cancer or a tumour.

11. Composition according to claim 10, for use in the treatment of a cancer of the cervix uteri, the ENT sphere or any other tumour induced by the HPV virus, of a liver cancer associated with chronic hepatitis B or C virus, or of a cancer expressing MUC1 protein.
